# EUROPEAN PATENT APPLICATION

(11) **EP 4 450 111 A1**
(43) Date of publication of application: **23.10.2024**
(21) Application number: 22907276.4
(22) Date of filing: 05.12.2022
(51) Int. Cl.: A61M 25/09

(54) **GUIDE WIRE**

(30) Priority: 17.12.2021 JP 2021205355
(71) Applicant: ASAHI INTECC CO., LTD., Seto-shi, Aichi, 489-0071 (JP)
(72) Inventor: ISHIKAWA Masatomo, Seto-shi, Aichi 489-0071 (JP); OTSUKA Yasunori, Seto-shi, Aichi 489-0071 (JP)
(74) Representative: Prinz & Partner mbB
(86) International application number: PCT/JP2022/044750
(87) International publication number: WO 2023/112748

(57) **Abstract**

A guide wire includes a core wire, wherein the core wire has a first magnetized region magnetized on the distal end side of the core wire, and the maximum value of the outer diameter of the first magnetized region is larger than the outer diameter of the distal end portion in an area located to the proximal end side of the first magnetized region.

## Description

### FIELD

The present invention relates to a guide wire.

### BACKGROUND

As a method for confirming the position of a medical device inserted into a living body lumen, a method using a contrast medium and radiography has been conventionally used, but in recent years, development of a technique for confirming the position using magnetism has been promoted. For example, Patent Literature 1 discloses a technique for detecting the magnetic region strength of a plurality of magnetic areas provided in a medical device to identify the position and orientation of the medical device. Patent Literature 2 discloses a technique for tracking the position of a guide wire around which a magnetic position sensor is wound based on magnetism. In addition, as another technique using magnetism, Patent Literature 3 discloses a technique for guiding a distal end portion by applying a magnetic repulsive force to a guide wire including a magnet head at the distal end portion.

### CITATION LIST

### Patent Literature

Patent Literature 1: JP 2019-520129 A
Patent Literature 2: JP 2020-108757 A
Patent Literature 3: JP 2013-103075 A

### SUMMARY OF THE INVENTION

### Technical Problem

All of the techniques disclosed in Patent Literature 1 to Patent Literature 3 are based on the premise that the source of magnetism has a certain degree of strong magnetism. Here, a case of magnetizing the distal end portion of a guide wire in order to track the behavior of the distal end portion is considered. In such a case, for example, as disclosed in Patent Literature 3, it is conceivable to arrange a permanent magnet at the distal end portion. However, this may lower the flexibility, and may complicate the structure of and increase the size of the distal end portion. Therefore, it is conceivable to magnetize the guide wire itself to have magnetism. However, the guide wire often has a thin diameter at such a distal end portion, so that even if the distal end portion is magnetized, the distal end portion may not be able to have strong magnetism as desired. In the techniques disclosed in Patent Literature 1 to Patent Literature 3, no consideration is given to magnetizing the distal end portion of the guide wire to have a desired strength of magnetism.

The present invention has been made to solve at least part of the above-mentioned problems, and an objective of the present invention is to provide a guide wire that can make it possible for a distal end portion to have a desired strength of magnetism.

### Solution to Problem

The present invention has been made to solve at least a part of the problems described above, and can be realized as the following aspects.

(1) According to one aspect of the present invention, a guide wire is provided. This guide wire is a guide wire including a core wire, wherein the core wire has a first magnetized region magnetized on the distal end side of the core wire, and the maximum value of the outer diameter of the first magnetized region is larger than the outer diameter of the distal end portion in the area located to the proximal end side of the first magnetized area.

According to this configuration, the maximum value of the outer diameter of the first magnetized region on the distal end side of the core wire is larger than the outer diameter of the distal end portion in an area located to the proximal end side of the first magnetized area. For this reason, compared to a core wire having a constant outer diameter on the distal end side or a tapered core wire having an outer diameter that decreases toward the distal end side, the volume of the first magnetized region per unit length can be increased, and thus the first magnetized region can have strong magnetism. Specifically, this enables the distal end portion of the guide wire to have a desired strength of magnetism. As a result, a magnetic sensor arranged outside the living body can easily detect the distal end portion of the guide wire inserted into a living body lumen, so that the detection accuracy of the magnetic sensor to detect the distal end portion of the guide wire can be improved. Moreover, such improved detection accuracy allows an operator to easily identify and track the movement of the distal end portion of the guide wire, so that the operator can perform the procedure more accurately and efficiently.

(2) In the guide wire of the above aspect, the core wire further has a second magnetized region magnetized at a position that is located to the proximal side of the first magnetized region and separated from the first magnetized region, and the maximum value of the outer diameter of the first magnetized region may be larger than the outer diameter of the distal end portion in the area between the first magnetized region and the second magnetized region.

According to this configuration, the core wire has the second region magnetized and located to the proximal end side of the first magnetized region, and the first magnetized region and the second magnetized region are separated. Therefore, the position of the first magnetized region and the position of the second magnetized region can be detected separately from each other. Also, the degree of curvature of the guide wire in the range from the second magnetized region to the first magnetized region can be determined based on the position of the first magnetized region and the position of the second magnetized region that are detected separately from each other.

(3) In the guide wire of the above aspect, the second magnetized region includes a magnetized coil that covers the outer peripheral surface of the core wire, and the maximum value of the outer diameter of the coil as the second magnetized region may be larger than the outer diameter of the proximal end portion in the area between the first magnetized region and the second magnetized region, and larger than the outer diameter of the distal end portion in an area located to the proximal end side of the second magnetized region.

According to this configuration, the volume of the second magnetized region per unit length can be increased while maintaining the flexibility of the core wire. Therefore, since the second magnetized region can have strong magnetism, accuracy for detecting the second magnetized region is improved. Thus, the degree of curvature of the guide wire in the range from the second magnetized region to the first magnetized area can be determined more accurately.

(4) In the guide wire of the above aspect, the core wire may be formed so that the maximum value of the outer diameter of the core wire in the second magnetized region is larger than the outer diameter of the proximal end portion in the area between the first magnetized region and the second magnetized region, and larger than the outer diameter of the distal end portion in the area located to the proximal end side of the second magnetized region.

According to this configuration, the volume of the second magnetized region per unit length can be increased without assembling another member to the core wire. Therefore, since the second magnetized region can have strong magnetism, accuracy for detecting the second magnetized region is improved. Thus, the degree of curvature of the guide wire in the range from the second magnetized region to the first magnetized region can be determined more accurately.

(5) In the guide wire of the above aspect, the core wire may have a curved portion in which the core wire is curved in the area between the first magnetized region and the second magnetized region.

According to this configuration, it is possible to improve the accuracy of detecting and distinguishing the first magnetized region provided to the distal end side of the curved portion and the second magnetized region provided to the proximal end side of the curved portion. With the use of the position of the first magnetized region and the position of the second magnetized region that are detected separately from each other, the three-dimensional movement of the distal end portion of the guide wire can be accurately identified and tracked. Specifically, when deformation such as bending occurs in the guide wire, a portion located to the distal end side of the curved portion mainly deforms. Therefore, through tracking of the position of the first magnetized region with reference to the position of the second magnetized region located to the proximal end side of the curved portion, the three-dimensional movement of the distal end portion of the guide wire can be accurately identified and tracked. As a result, the operator can perform the procedure more accurately and efficiently.

(6) In the guide wire of the above aspect, the proximal end portion of the first magnetized region may be formed with an enlarged diameter portion having an outer diameter that gradually increases from the proximal end side to the distal end side.

This configuration makes it difficult to create a rigidity gap with which the bending rigidity is greatly varied at the proximal end portion of the first magnetized region, so as to be able to suppress the bending of the proximal end portion of the first magnetized area, and increase the volume of the first magnetized region per unit length.

(7) In the guide wire of the above aspect, the distal end portion of the first magnetized region may also be formed with a distal end reduced diameter portion having an outer diameter that gradually decreases from the proximal end side to the distal end side.

According to this configuration, since the outer diameter of the distal end portion of the first magnetized region gradually decreases from the proximal end side to the distal end side, it is possible to facilitate the entry of the guide wire into a lesion or the like, despite the presence of the lesion or the like that clogs a living body lumen into which the guide wire is inserted.

(8) According to one aspect of the present invention, a guide wire is provided. The guide wire is a guide wire including a core wire and a coil covering the core wire, wherein the coil has a magnetized region that is magnetized, and a wire forming the magnetized region of the coil is thicker than a wire forming the distal end portion in the area located to the proximal end side of the magnetized region.

According to this configuration, the wire forming the magnetized region is thicker than the wire forming the distal end portion in the area located to the proximal end side of the magnetized region. Therefore, the volume of the magnetized region per unit length can be increased, so that the magnetized region can have strong magnetism.

The present invention can be implemented in various aspects, for example, a core wire to be used for a guide wire, a medical device including a guide wire, a method for producing a core wire or a guide wire, and a system for detecting the position of a guide wire that is inserted into the body.

### BRIEF DISCRIPTION OF DRAWINGS

FIG. 1 is an explanatory view that schematically shows the configuration of a guide wire of a first embodiment.
FIGS. 2A and 2B are transverse sectional views of the guide wire of the first embodiment.
FIG. 3 is a flow chart of a method for producing the guide wire of the first embodiment.
FIGS. 4A to 4C are explanatory views that show the production steps of the guide wire of the first embodiment.
FIG. 5 is an explanatory view that schematically shows the configuration of a guide wire of a second embodiment.
FIGS. 6A to 6C are transverse sectional views of the guide wire of the second embodiment.
FIGS. 7A to 7D are explanatory views that show the production steps of the guide wire of the second embodiment.
FIG. 8 is an explanatory view that schematically shows the configuration of a guide wire of a third embodiment.
FIG. 9 is an explanatory view that schematically shows the configuration of a guide wire of a fourth embodiment.
FIGS. 10A to 10C are transverse sectional views of the guide wire of the fourth embodiment.
FIG. 11 is an explanatory view that schematically shows the configuration of a guide wire of a fifth embodiment.
FIG. 12 is an explanatory view that schematically shows the configuration of a guide wire of a sixth embodiment.
FIG. 13 is an explanatory view that schematically shows the configuration of a guide wire of a seventh embodiment.
FIGS. 14A to 14C are explanatory views that schematically show the configurations of guide wires of the eighth to tenth embodiments.

### DESCRIPTION OF EMBODIMENTS

### <First Embodiment>

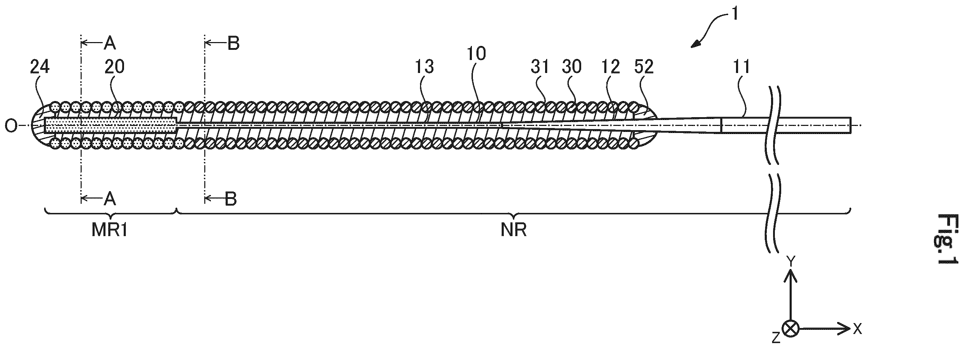

FIG. 1 is an explanatory view that schematically shows the configuration of a guide wire 1 according to a first embodiment. The guide wire 1 is a medical device that is inserted into a living body lumen in each organ of a human body, such as blood vessels, the lymph gland system, the biliary system, the urinary tract system, the airway system, the digestive organ system, secretory glands, and reproductive organs. The guide wire 1 may be inserted directly into the above-described living body lumen, or may be inserted into the living body lumen via an endoscope. The guide wire 1 includes a core wire 10, a distal end joint part 24, an outer coil 30 and a proximal end joint part 52.

In FIG. 1, the axis passing through the center of the guide wire 1 is represented by an axis O (one-dot chain line). FIG. 1 shows a longitudinal section of the guide wire 1 along the axis O direction. In an example of FIG. 1, the axis O coincides with the axis passing through each center of the guide wire 1 and each component. However, the axis O may be different from each central axis of the guide wire 1 and each component. Fig. 1 illustrates XYZ axes that are orthogonal to each other. The X-axis corresponds to the longitudinal direction of the guide wire 1 (the direction of the axis O), the Y-axis corresponds to the height direction of the guide wire 1, and the Z-axis corresponds to the width direction of the guide wire 1. The left side (-X-axis direction) in FIG. 1 is referred to as the "distal end side" of the guide wire 1 and that of each component, while the right side (+X-axis direction) in FIG. 1 is referred to as the "proximal end side" of the guide wire 1 and that of each component. Further, regarding the guide wire 1 and each component, the end part located on the distal end side is referred to as a "distal end", and the distal end and its vicinity are referred to as a "distal end portion". Further, the end part located on the proximal end side is referred to as a "proximal end", and the proximal end and its vicinity are referred to as a "proximal end portion". The portion on the distal end side is inserted into a living body, and the portion on the proximal end side is operated by an operator such as a doctor. These points are also common to FIG. 1 and the subsequent figures.

The core wire 10 is an elongated member. The core wire 10 has a large diameter portion 11, a reduced diameter portion 12, a small diameter portion 13, and a distal end large diameter portion 20 in order from the proximal end side to the distal end side.

The large diameter portion 11 is formed on the proximal end side of the core wire 10. The large diameter portion 11 has a substantially cylindrical shape with a substantially constant outer diameter, and is a portion having a larger outer diameter than that of the reduced diameter portion 12 and that of the small diameter portion 13. As used herein, "substantially constant" is synonymous with "generally constant", and means to be generally constant while allowing fluctuations due to manufacturing errors or the like. The large diameter portion 11 is not covered with an outer coil 30 and is used when an operator holds the guide wire 1. The reduced diameter portion 12 is formed between the large diameter portion 11 and the small diameter portion 13 of the core wire 10. The reduced diameter portion 12 has a tapered shape in which the outer diameter gradually decreases from the proximal end side to the distal end side. The small diameter portion 13 is formed between the distal end large diameter portion 20 and the reduced diameter portion 12 of the core wire 10. The small diameter portion 13 has a substantially cylindrical shape with a substantially constant outer diameter, and is a portion with the smallest outer diameter of the core wire 10. The outer diameter and the length of each portion (the large diameter portion 11, the reduced diameter portion 12, and the small diameter portion 13) of the core wire 10 can be arbitrarily determined.

The distal end large diameter portion 20 is formed on the distal end side of the core wire 10. The distal end large diameter portion 20 has a substantially cylindrical shape with a substantially constant outer diameter. Further, the distal end large diameter portion 20 is the first magnetized region MR1 magnetized on the distal end side of the core wire 10. Here, the magnetized region is a region magnetized by applying an external magnetic field. In FIG. 1, a dot pattern is formed by a hatching technique on the distal end large diameter portion 20 and the portion of the outer coil 30 arranged therearound, indicating the magnetized portions. The same applies to FIG. 1 and the subsequent figures such that the dot pattern formed by the hatching technique indicates magnetized portions. In the present embodiment, non-magnetized regions (the small diameter portion 13, the reduced diameter portion 12, and the large diameter portion 11) located to the proximal end side of the first magnetized region MR1 of the core wire 10 correspond to the non-magnetized region(s) NR. Here, the non-magnetized region is a region that is not magnetized by applying an external magnetic field, but may be slightly magnetized due to the influence of shape processing or the like.

The material forming the distal end large diameter portion 20 can be arbitrarily determined as long as it is a magnetic material that is magnetized by applying an external magnetic field. Examples of such materials include stainless steel undergoing martensitic transformation (e.g., SUS304, SUS316, SUS302, SUS444, SUS434, and SUS630), martensitic stainless steel (e.g., SUS410), and ferritic stainless steel (e.g., SUS430). Meanwhile, the material forming the portion of the core wire 10, which corresponds to the non-magnetized region NR, may be the same material as that of the distal end large diameter portion 20, or may be a non-magnetic material.

The distal end joint part 24 joins the outer coil 30 and the distal end of the core wire 10 (the distal end of the distal end large diameter portion 20). The proximal end joint part 52 joins the outer coil 30 and the reduced diameter portion 12 of the core wire 10. For joining at the distal end joint part 24 and the proximal end joint part 52, any adhesive such as a metal solder, e.g., silver brazing, gold brazing, zinc, Sn-Ag alloy, and Au-Sn alloy, and an epoxy adhesive can be used.

The outer coil 30 has a substantially cylindrical shape with a substantially constant outer diameter from the proximal end side to the distal end side. The outer coil 30 is arranged so as to partially cover the distal end large diameter portion 20, the small diameter portion 13 and the reduced diameter portion 12 of the core wire 10. In this embodiment, the outer coil 30 is a single-thread coil formed by winding one wire 31 in a single thread. Note that the outer coil 30 may be a multi-thread coil formed by winding a plurality of wires in multiple threads, and may be a single-thread twisted wire coil formed by winding a twisted wire obtained by twisting a plurality of wires, in a single thread, or a multi-thread twisted wire coil formed by winding each twisted wire in multiple threads with the use of a plurality of twisted wires obtained by twisting a plurality of wires. The outer diameter and the inner diameter of the outer coil 30 can be determined arbitrarily. Further, in the present embodiment, the outer coil 30 is formed of a magnetic material that is magnetized by applying an external magnetic field, as in the case of the distal end large diameter portion 20, however, in another embodiment, the outer coil 30 may be formed of a non-magnetic material.

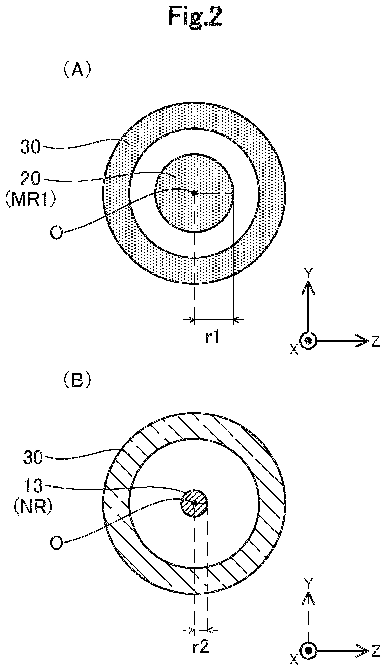

FIGS. 2A and 2B are transverse sectional views of the guide wire 1. FIG. 2A shows a transverse section at line A-A in FIG. 1. FIG. 2A shows a transverse section at the position of the distal end large diameter portion 20 of the core wire 10. The outer diameter r1 is the outer diameter of the distal end large diameter portion 20 that is the first magnetized region MR1. FIG. 2B shows a transverse section at line B-B in FIG. 1. FIG. 2B shows a transverse section at the position of the small diameter portion 13 of the core wire 10. The outer diameter r2 is the outer diameter of the small diameter portion 13 that is a non-magnetized region NR. As shown in FIGS. 2A and 2B, the maximum value of the outer diameter r1 of the first magnetized region MR1 is larger than the outer diameter r2 of the distal end portion (the small diameter portion 13) in the area (non-magnetized region NR) located to the proximal end side of the first magnetized region MR1. In the present embodiment, both the distal end large diameter portion 20 and the small diameter portion 13 are substantially cylindrical and have a circular transverse section, so that the outer diameters r1 and r2 are uniquely determined. However, when the transverse section of the distal end large diameter portion 20 and the same of the small diameter portion 13 are elliptical, the length of the longest portion of an arbitrary transverse section is defined as the outer diameter. Further, when the distal end large diameter portion 20 has a shape different from the substantially cylindrical shape (a shape in which the cross-sectional area of the transverse section is not substantially constant), the longest outer diameter r1 among the outer diameters r1 of transverse sections is defined as the maximum value and this is compared with the outer diameter r2. Similarly, when the distal end portion of the non-magnetized region NR has a shape different from the substantially cylindrical shape (a shape in which the cross-sectional area of the transverse section is not substantially constant), the longest outer diameter r2 among the outer diameters r2 of transverse sections is compared with the maximum value of the outer diameter r1.

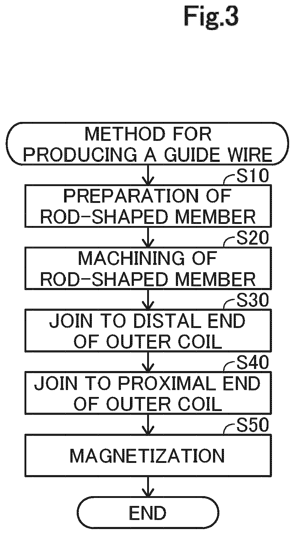

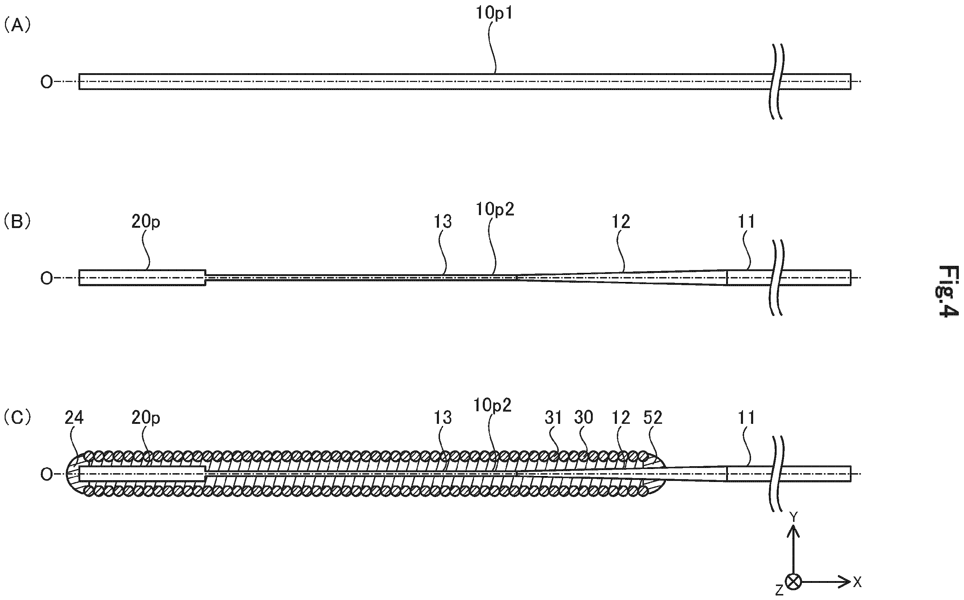

FIG. 3 is a flow chart of the method for producing the guide wire 1. FIGS. 4A to 4C are explanatory views that show the production steps of the guide wire 1. The XYZ axes illustrated at the lower right position in FIG. 4 are common to FIGS. 4A, 4B and 4C. In production of the guide wire 1, as shown in FIG. 4A, first, a rod-shaped member 10p1 is prepared (step S10). The rod-shaped member 10p1 is a base member of the core wire 10 (FIG. 1). Next, as shown in FIG. 4B, a rod-shaped member 10p2 is formed by machining the rod-shaped member 10p1 (step S20). Machining is performed using a grinder or the like. The rod-shaped member 10p2 has a large diameter portion 11, a reduced diameter portion 12, a small diameter portion 13, and a cylindrical portion 20p in order from the proximal end side to the distal end side. The cylindrical portion 20p is a base member of the distal end large diameter portion 20, and is the distal end large diameter portion 20 before being magnetized. Specifically, the rod-shaped member 10p2 has the shape of a core wire 10, but is a core wire 10 having a non-magnetized distal end portion.

Next, as shown in FIG. 4C, the distal end of the cylindrical portion 20p is joined to the distal end of the outer coil 30 via the distal end joint part 24 (step S30), and a portion of the reduced diameter portion 12 is joined to the proximal end of the outer coil 30 via the proximal end joint part 52 (step S40). Subsequently, the cylindrical portion 20p is magnetized by applying an external magnetic field from a magnetizing device (not shown) to be the distal end large diameter portion 20 (step S50). At this time, a portion of the outer coil 30 arranged around the cylindrical portion 20p is also magnetized (FIG. 1). The magnetizing device may include, for example, a permanent magnet or an air-core coil that generates a magnetic field by applying an electric current. After application of the external magnetic field, the method for producing the guide wire 1 is completed. In addition, although the shape of the core wire 10 is integrally formed in the production steps described with reference to FIGS. 4A to 4C, it may be formed separately. In such a case, a rod-shaped member having a large diameter portion 11, a reduced diameter portion 12, and a small diameter portion 13 is produced by machining, and then a cylindrical portion 20p (the base member of the distal end large diameter portion 20) is joined to the distal end of the rod-shaped member, thereby forming the shape of the core wire 10. Joining is performed by laser welding or soldering.

As described above, according to the guide wire 1 of the first embodiment, the volume per unit length of the distal end large diameter portion 20 (the first magnetized area MR1) formed at the distal end portion of the core wire 10 can be increased compared with a core wire having a constant outer diameter on the distal end side and a tapered core wire having an outer diameter that decreases toward the distal end side. Accordingly, the distal end large diameter portion 20 (first magnetized region MR1) can have strong magnetism (Note that the "unit length" used herein refers to the unit length in the longitudinal direction of the core wire 10.). Specifically, this enables the distal end portion of the guide wire 1 to have a desired strength of magnetism. As a result, a magnetic sensor arranged outside the living body can easily detect the distal end portion of the guide wire 1 inserted into a living body lumen, so that the detection accuracy of the magnetic sensor to detect the distal end portion of the guide wire 1 can be improved. Moreover, improved detection accuracy allows an operator to easily identify and track the movement of the distal end portion of the guide wire 1, so that the operator can perform the procedure more accurately and efficiently.

Further, in the present embodiment, the distal end large diameter portion 20 (first magnetized region MR1) is formed to have a substantially cylindrical shape, so as to increase the volume constituted by the distal end large diameter portion 20 (the first magnetized region MR1) in the internal space of the substantially cylindrical outer coil 30 as large as possible. This can increase the magnetism the distal end large diameter portion 20 (the first magnetized region MR1) can have. Meanwhile, the outer diameter of the distal end portion of the non-magnetized region NR is smaller than the maximum value of the outer diameter r1 of the distal end large diameter portion 20, so that the bending rigidity of the core wire 10 located to the proximal side of the distal end large diameter portion 20 can be reduced. Therefore, the bendability of the guide wire 1 is maintained.

When such a guide wire 1 of the first embodiment is inserted into a living body lumen, while the behavior of the guide wire 1 as a whole is identified and tracked by a conventional method using a contrast medium and radiography, detailed behavior of the distal end portion of the guide wire 1 can be identified and tracked with a magnetic sensor.

### <Second Embodiment>

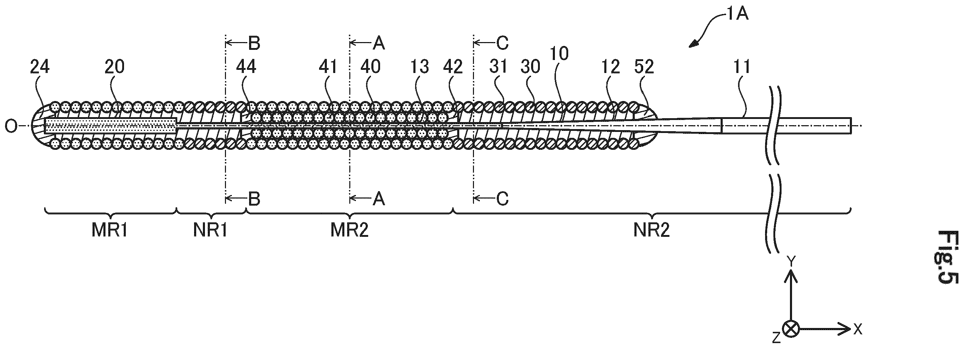

FIG. 5 is an explanatory view that schematically shows the configuration of the guide wire 1A of a second embodiment. The guide wire 1A of the second embodiment differs from the guide wire 1 of the first embodiment (FIG. 1) in that it includes an inner coil 40, a proximal joint part 42, and a distal joint part 44.

The inner coil 40 has a substantially cylindrical shape with a substantially constant outer diameter from the proximal end side to the distal end side. The inner coil 40 is a magnetized coil that covers the outer peripheral surface of the core wire 10 (small diameter portion 13). The inner coil 40 is joined to the core wire 10 (small diameter portion 13) and the outer coil 30 via the proximal joint part 42 and the distal joint part 44. Note that the inner coil 40 does not have to be joined to the outer coil 30. Moreover, the inner coil 40 is a single thread coil formed by winding one wire 41 into a single thread as in the case of the outer coil 30, but may also be a multi-thread coil, a single-thread twisted wire coil, or a multi-thread twisted wire coil. Further, the inner coil 40 is made of a magnetic material that is magnetized by applying an external magnetic field, as in the case of the distal end large diameter portion 20 and the outer coil 30.

As shown in FIG. 5, in the second embodiment, in addition to the fact that the distal end large diameter portion 20 and the portion of the outer coil 30 arranged therearound are magnetized, the inner coil 40, a coated portion of the core wire 10, the outer peripheral surface of which is covered with the inner coil 40, and a portion of the outer coil 30 corresponding to a position where the inner coil 40 is present in the X-axis direction are also magnetized. Specifically, in the second embodiment, the core wire 10 has the second magnetized region MR2 (FIG. 5) magnetized at a position that is located to the proximal end side of the first magnetized region MR1 and separated from the first magnetized region MR1. The second magnetized region MR2 includes a covered portion of the core wire 10, which is covered with the inner coil 40, and the inner coil 40. Moreover, the first non-magnetized region NR1 is provided between the first magnetized region MR1 and the second magnetized region MR2, and a second non-magnetized region NR2 is provided to the proximal end side of the second magnetized region MR2. A portion located to the distal end side of the coated portion of the core wire 10, which is covered with the inner coil 40, corresponds to the first non-magnetized region NR1, and a portion located to the proximal end side of the covered portion corresponds to the second non-magnetized region NR2.

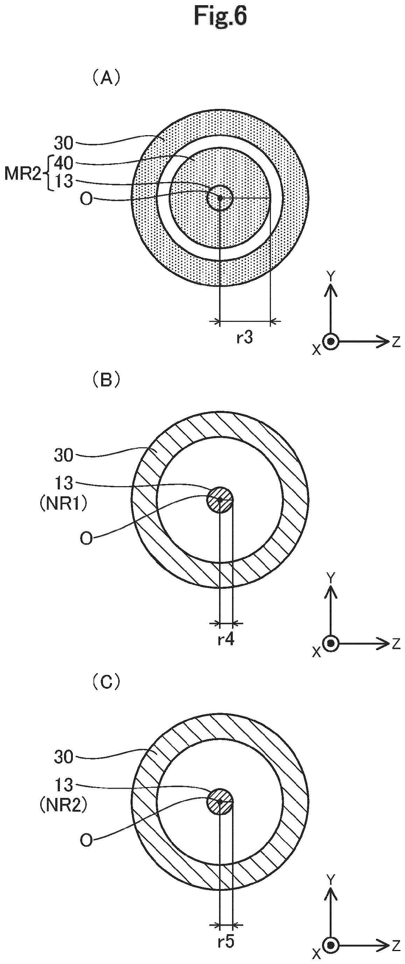

FIGS. 6A to 6C are transverse sectional views of a guide wire 1A. FIG. 6A shows a transverse section at line A-A in FIG. 5. FIG. 6B shows a transverse section at line B-B on the proximal end portion of the first non-magnetized region NR1 in FIG. 5. FIG. 6C shows a transverse section at line C-C on the distal end portion of the second non-magnetized region NR2 in FIG. 5. In FIG. 6A, the outer diameter r3 is the outer diameter of the second magnetized region MR2 and is the outer diameter of the inner coil 40 as well. In FIG. 6B, the outer diameter r4 is the outer diameter of the first non-magnetized region NR1 and is the outer diameter of the small diameter portion 13 as well. In FIG. 6C, the outer diameter r5 is the outer diameter of the second non-magnetized region NR2 and is the outer diameter of the small diameter portion 13 as well. As shown in FIGS. 6A, 6B and 6C, the maximum value of the outer diameter r3 of the inner coil 40 as the second magnetized region MR2 is larger than the outer diameter r4 of the proximal end portion of the first non-magnetized region NR1, and is also larger than the outer diameter r5 of the distal end portion of the second non-magnetized region NR2. Note that, as in the case of the first embodiment, if there is a region having a shape in which the cross-sectional area of the transverse section is not substantially constant among the second magnetized region MR2 and the first and second non-magnetized regions NR1 and NR2, the longest outer diameter among the outer diameters of transverse sections within the regions shall be used for comparison with the outer diameters of other regions.

Further, the distal end portion of the non-magnetized region in the guide wire 1 of the first embodiment (the position at line B-B in FIG. 1 and its vicinity) corresponds to the distal end portion of the first non-magnetized region NR1 in the guide wire 1A of the second embodiment. Therefore, it can be said that the maximum value of the outer diameter r1 (FIG. 2A) of the distal end large diameter portion 20 is larger than the outer diameter r4 (FIGS. 6B) of the distal end portion of the first non-magnetized region NR1.

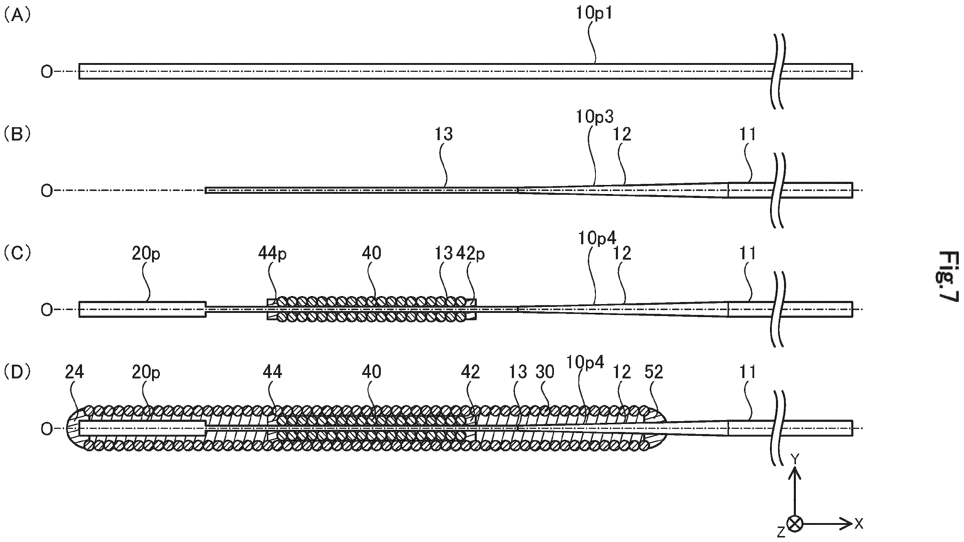

Next, the method for producing the guide wire 1A is described. FIGS. 7A to 7D are explanatory views that show the production steps of the guide wire 1A. The XYZ axes illustrated at the lower right position in FIG. 7 are common to FIGS. 7A to 7D. The method for producing the guide wire 1A is similar to the flow chart shown in FIG. 3 and is described below. First, as shown in FIG. 7A, after preparing the rod-shaped member 10p1 (corresponding to step S10 in FIG. 3), as shown in FIG. 7B, the rod-shaped member 10p1 is machined to form a rod-shaped member 10p3 (corresponding to step S20 in FIG. 3). The rod-shaped member 10p3 has a large diameter portion 11, a reduced diameter portion 12, and a small diameter portion 13 in order from the proximal end side to the distal end side. Next, as shown in FIG. 7C, from the distal end side of the rod-shaped member 10p3, the inner coil 40 is arranged at the position of the small diameter portion 13 and joined via the joint part 42p and the joint part 44p, and then the cylindrical portion 20p is joined to the distal end of the rod-shaped member 10p3, thereby forming a rod-shaped member 10p4. Joining at this time is also performed by laser welding or soldering. Next, as shown in FIG. 7D, the distal end of the cylindrical portion 20p is joined to the distal end of the outer coil 30 via the distal end joint part 24 (corresponding to step S30 in FIG. 3). Next, a bonding agent is poured from the outside of the outer coil 30, so that the small diameter portion 13 and the inner coil 40 are joined to an intermediate portion of the outer coil 30 via the proximal joint part 42 and the distal joint part 44 formed by expanding a joint part 42p and a joint part 44p. Next, a portion of the reduced diameter portion 12 is joined to the proximal end of the outer coil 30 via the proximal end joint part 52 (corresponding to step S40 in FIG. 3). Thereafter, the cylindrical portion 20p, the covered portion of the small diameter portion 13, which is covered by the inner coil 40, and the inner coil 40 are magnetized by applying an external magnetic field from a magnetizing device (not shown) (corresponding to step S50 in FIG. 3). At this time, a portion of the outer coil 30, which is arranged around the cylindrical portion 20p and around the inner coil 40, is also magnetized. Note that in the above-described series of steps, when a step of joining the small diameter portion 13 and the inner coil 40 to an intermediate portion of the outer coil 30 is omitted, a guide wire in which the inner coil 40 is not joined to the outer coil 30 can also be produced.

Also with the guide wire 1A of the second embodiment as described above, it becomes possible for the distal end portion of the guide wire 1A to have a desired strength of magnetism, as in the case of the first embodiment. Further, according to the guide wire 1A of the second embodiment, the first magnetized region MR1 and the second magnetized region MR2 are separated by the first non-magnetized region NR1, so that the position of the first magnetized region MR1 and the position of the second magnetized region MR2 can be detected separately from each other. Also, the degree of curvature of the guide wire 1A in the range from the second magnetized region MR2 to the first magnetized region MR1 can be determined based on the position of the first magnetized region and the position of the second magnetized region that are detected separately from each other.

In the guide wire 1A of the second embodiment, the maximum value of the outer diameter r3 of the inner coil 40 as the second magnetized region MR2 is larger than the outer diameter r4 of the proximal end portion of the first non-magnetized region NR1, and, is also larger than the outer diameter r5 of the distal end portion of the second non-magnetized region NR2. Therefore, the volume of the second magnetized region MR2 per unit length can be increased while maintaining the flexibility of the core wire 10. Accordingly, the second magnetized region MR2 can have strong magnetism, so that accuracy for detecting the second magnetized region MR2 is improved. Thus, the degree of curvature of the guide wire 1A in the range from the second magnetized region MR2 to the first magnetized region MR1 can be determined more accurately.

When such a guide wire 1A of the second embodiment is inserted into a living body lumen, through identifying and tracking of the behavior of the guide wire 1A in the range from the second magnetized region MR2 to the first magnetized region MR1 in real time by a magnetic sensor and through identifying and tracking of the same by a conventional method using a contrast medium and radiography at regular timing, the amount of radiation exposure due to radiography can be suppressed more effectively than in a case of identifying and tracking the behavior using only such a conventional method for identifying and tracking the behavior.

In the guide wire 1A of the second embodiment, when the first magnetized region MR1 has magnetism stronger than that of the second magnetized region MR2, the procedure, for which identifying and tracking of the behavior of the distal end portion of the guide wire 1A is important, can be performed more accurately and efficiently. Further, when the first magnetized region MR1 and the second magnetized region MR2 have magnetism equivalent to each other, the strength of the magnetism detected by the magnetic sensor from each region is proportional to the distance from each region. Hence, the guide wire 1A with which the position of each region can be most easily determined in terms of software. Further, when the second magnetized region MR2 has stronger magnetism than that of the first magnetized region MR1, the position of the second magnetized region MR2 can be easily determined. For example, if the distal end portion of the guide wire 1A is inserted in advance into one blood vessel on the far side from a branch position so that the second magnetized region MR2 is arranged at the branch position where the blood vessel branches, the second magnetized region MR2 can be used as a marker indicating the branch position, when another guide wire is inserted into the other blood vessel on the far side from the branch position. It should be noted that even when another guide wire is being operated, whether or not the position of the previously inserted guide wire 1A is misaligned can be determined in real time by the magnetic sensor.

### <Third Embodiment>

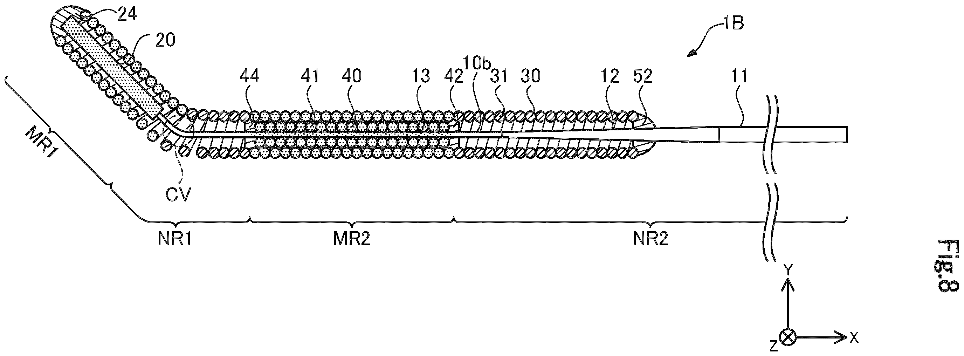

FIG. 8 is an explanatory view that schematically shows the configuration of a guide wire 1B of a third embodiment. The guide wire 1B of the third embodiment differs from the guide wire 1A (FIG. 5) of the second embodiment in that it includes a core wire 10b having a curved portion CV.

In the third embodiment, the core wire 10b has a curved portion CV at which the core wire 10b is curved in the first non-magnetized region NR1, which is the area between the first magnetized region MR1 and the second magnetized region MR2. The curved portion CV is also a portion where the axis of the guide wire 1B extending in a certain direction from the proximal end side is curved. Since the core wire 10b has the curved portion CV, the operability of the distal end portion of the guide wire 1B is improved. For example, when the guide wire 1B is inserted into a blood vessel, blood vessel selectivity is improved.

Also with the guide wire 1B of the third embodiment as described above, it becomes possible for the distal end portion of the guide wire 1B to have a desired strength of magnetism, as in the case of the first embodiment. According to the guide wire 1B of the third embodiment, it is possible to improve the accuracy of detecting the first magnetized region MR1 provided on the distal end side of the curved portion CV and the second magnetized region MR2 provided to the proximal end side of the curved portion CV separately from each other. With the use of the position of the first magnetized region MR1 and the position of the second magnetized region MR2 that are detected separately from each other, the three-dimensional movement of the distal end portion of the guide wire 1B can be identified and tracked more accurately. Specifically, when deformation such as bending occurs in the guide wire 1B, a portion located to the distal end side of the curved portion mainly deforms. Therefore, through tracking of the position of the first magnetized region MR1 with reference to the position of the second magnetized region MR2 located to the proximal end side of the curved portion CV, the three-dimensional movement of the distal end portion of the guide wire 1B can be identified and tracked more accurately. As a result, the operator can perform the procedure more accurately and efficiently.

### <Fourth Embodiment>

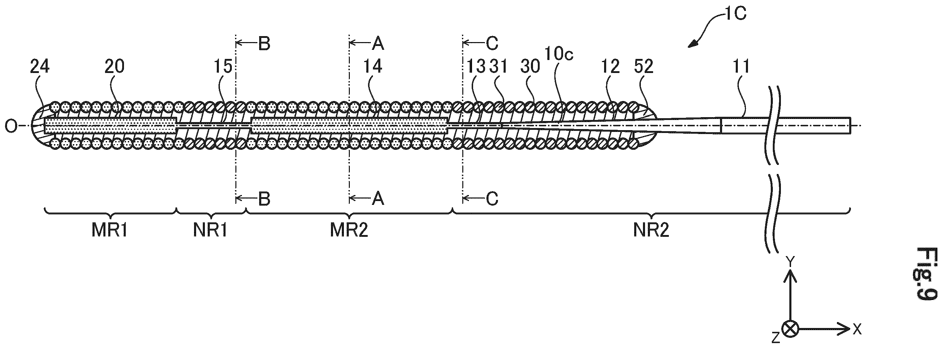

FIG. 9 is an explanatory view that schematically shows the configuration of a guide wire 1C of a fourth embodiment. The guide wire 1C of the fourth embodiment differs from the guide wire 1A (FIG. 5) of the second embodiment in that it does not include the inner coil 40, but includes a core wire 10c having an intermediate large diameter portion 14 and a distal end side small diameter portion 15.

The intermediate large diameter portion 14 is formed at the distal end of the small diameter portion 13. The intermediate large diameter portion 14 has a substantially cylindrical shape with a substantially constant outer diameter, and is a portion having a larger outer diameter than that of the small diameter portion 13 or the distal end side small diameter portion 15 formed at the distal end of the intermediate large diameter portion 14. In FIG. 9, a dot pattern is formed by the hatching technique on the intermediate large diameter portion 14 and the portion of the outer coil 30 covering the periphery of the intermediate large diameter portion 14 in the X-axis direction, indicating the state of being magnetized. Specifically, in the fourth embodiment, the intermediate large diameter portion 14 is the second magnetized region MR2 that is magnetized and located to the proximal side of the first magnetized region MR1. Note that in the fourth embodiment, a distal end large diameter portion 20 is formed on the distal end side of the distal end side small diameter portion 15.

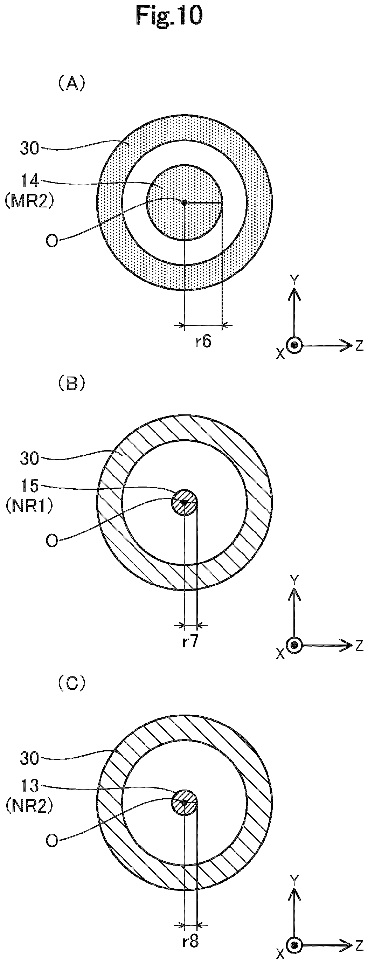

FIGS. 10A to 10C are transverse sectional views of a guide wire 1C. FIG. 10A shows a transverse section at line A-A in FIG. 9. FIG. 10B shows a transverse section at line B-B on the proximal end portion of the first non-magnetized region NR1 in FIG. 9. FIG. 10C shows a transverse section at line C-C on the distal end portion of the second non-magnetized region NR2 in FIG. 9. In FIG. 10A, an outer diameter r6 is the outer diameter of the second magnetized region MR2 and is the outer diameter of the intermediate large diameter portion 14 as well. In FIG. 10B, an outer diameter r7 is the outer diameter of the first non-magnetized region NR1 and is the outer diameter of the distal end side small diameter portion 15 as well. In FIG. 10C, an outer diameter r8 is the outer diameter of the second non-magnetized region NR2 and is the outer diameter of the small diameter portion 13 as well. As shown in FIGS. 10A, 10B and 10C, the core wire 10c is formed, so that the maximum value of the outer diameter r6 of the core wire 10c (intermediate large diameter portion 14) in the second magnetized region MR2 is larger than the outer diameter r7 in the proximal end portion of the first non-magnetized region NR1, and, larger than the outer diameter r8 in the distal end portion of the second non-magnetized region NR2. Note that, as in the case of the second embodiment (FIG. 5), if there is a region having a shape in which the cross-sectional area of the transverse section is not substantially constant among the second magnetized region MR2 and the first and second non-magnetized regions NR1 and NR2, the longest outer diameter among the outer diameters of transverse sections within the regions shall be used for comparison with the outer diameters of other regions.

Also with the guide wire 1C of the fourth embodiment as described above, it becomes possible for the distal end portion of the guide wire 1C to have a desired strength of magnetism, as in the case of the first embodiment. Moreover, the volume of the second magnetized region MR2 per unit length can be increased without assembling another member to the core wire 10c. Therefore, since the second magnetized region MR2 can have strong magnetism, as in the case of the second embodiment, accuracy for detecting the second magnetized region MR2 is improved. Thus, the degree of curvature of the guide wire 1C in the range from the second magnetized region MR2 to the first magnetized region MR1 can be determined more accurately.

### <Fifth Embodiment>

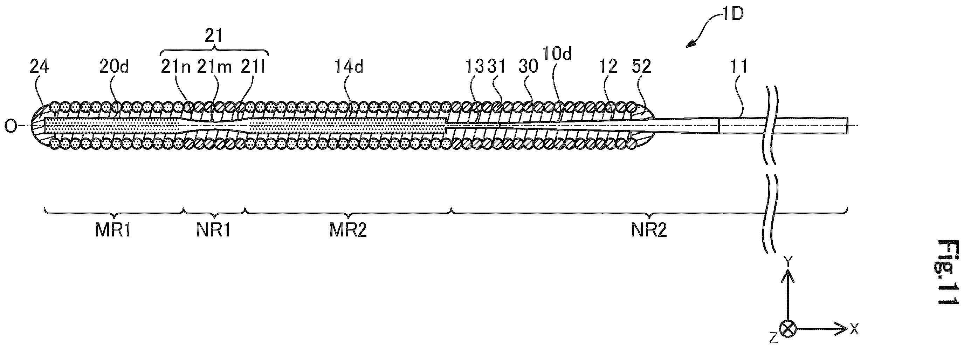

FIG. 11 is an explanatory view that schematically shows the configuration of a guide wire 1D of the fifth embodiment. The guide wire 1D of the fifth embodiment mainly differs from the guide wire 1C (FIG. 9) of the fourth embodiment in that it includes a core wire 10d having a constricted portion 21.

The constricted portion 21 is formed between an intermediate large diameter portion 14d and a distal end large diameter portion 20d. The constricted portion 21 has a reduced diameter portion 211, an intermediate small diameter portion 21m, and an enlarged diameter portion 21n in order from the proximal end side to the distal end side. The reduced diameter portion 211 is formed in the proximal end portion of the constricted portion 21. In other words, the reduced diameter portion 211 is formed in the distal end portion of the second magnetized region MR2. The reduced diameter portion 211 is a portion having an outer diameter that gradually decreases from the proximal end side to the distal end side. The intermediate small diameter portion 21m is formed between the reduced diameter portion 21l and the enlarged diameter portion 21n of the constricted portion 21. The intermediate small diameter portion 21m is a portion having an outer diameter that gradually decreases toward the central position in the X-axis direction. The enlarged diameter portion 21n is formed in the distal end portion of the constricted portion 21. In other words, the enlarged diameter portion 21l is formed in the proximal end portion of the first magnetized region MR1. The enlarged diameter portion 21n is a portion having an outer diameter that gradually increases from the proximal end side to the distal end side.

Also with the guide wire 1D of the fifth embodiment as described above, it becomes possible for the distal end portion of the guide wire 1D to have a desired strength of magnetism, as in the case of the first embodiment. Moreover, in the fifth embodiment, the proximal end portion of the first magnetized region MR1 may be formed with an enlarged diameter portion 21n having an outer diameter that gradually increases from the proximal end side to the distal end side. Therefore, by making it difficult to create a rigidity gap with which the bending rigidity is greatly varied in the proximal end portion of the first magnetized region MR1, the bending of the proximal end portion of the first magnetized region MR1 can be suppressed, and the volume of the first magnetized region MR1 can be increased. Further, in the fifth embodiment, since the reduced diameter portion 211 is formed in the distal end portion of the second magnetized region MR2, bending of the distal end portion of the second magnetized region MR2 can also be suppressed.

### <Sixth Embodiment>

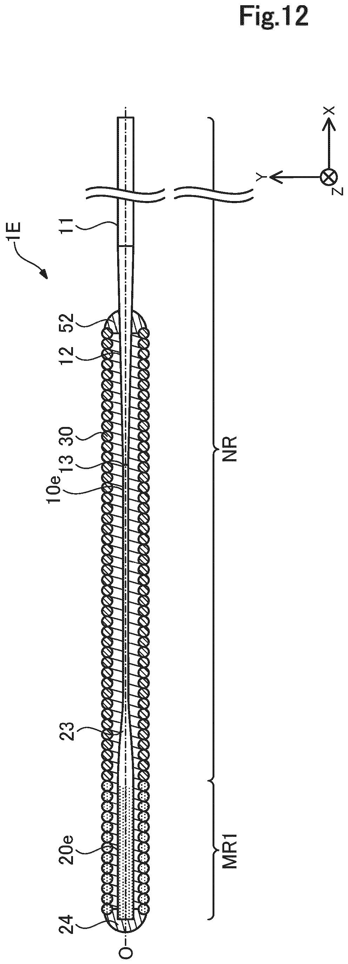

FIG. 12 is an explanatory view that schematically shows the configuration of the guide wire 1E of a sixth embodiment. The guide wire 1E of the sixth embodiment mainly differs from the guide wire 1D (FIG. 11) of the fifth embodiment in that it includes a core wire 10e having no intermediate large diameter portion 14d.

In the core wire 10e, a distal end large diameter portion 20e is formed at the distal end of the enlarged diameter portion 23 formed at the distal end of the small diameter portion 13. The enlarged diameter portion 23 is formed in the proximal end portion of the first magnetized region MR1 as in the case of the enlarged diameter portion 21n (FIG. 11), and the outer diameter of which gradually increases from the proximal end side to the distal end side. Note that in the sixth embodiment, non-magnetized portions (the enlarged diameter portion 23, the small diameter portion 13, the reduced diameter portion 12 and the large diameter portion 11) located to the proximal end side of the first magnetized region MR1 of the core wire 10e correspond to the non-magnetized region NR.

Also with the guide wire 1E of the sixth embodiment as described above, it becomes possible for the distal end portion of the guide wire 1E to have a desired strength of magnetism, as in the case of the first embodiment. Moreover, by making it difficult to create a rigidity gap with which the bending rigidity is greatly varied in the proximal end portion of the first magnetized region MR1, the bending of the proximal end portion of the first magnetized region MR1 can be suppressed, and the volume of the first magnetized region MR1 per unit length can be increased.

### <Seventh Embodiment>

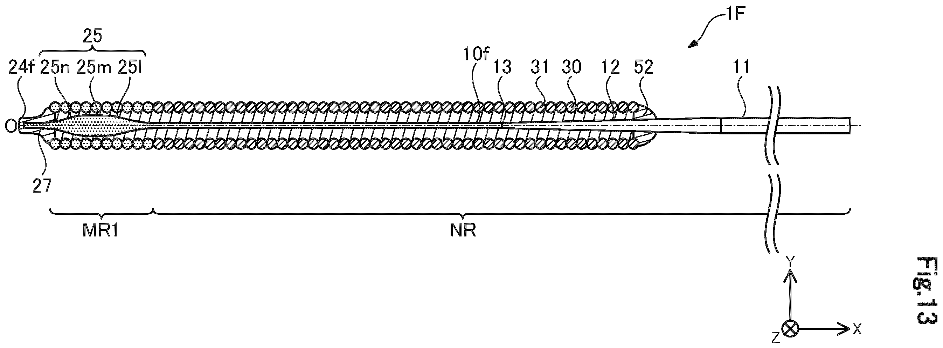

FIG. 13 is an explanatory view that schematically shows the configuration of the guide wire 1F of a seventh embodiment. The guide wire 1F of the seventh embodiment differs from the guide wire 1E (FIG. 12) of the seventh embodiment in that it includes a core wire 10f differing from the core wire 10e in the shape on the distal end side.

The core wire 10f has an expansion portion 25 and a distal end reduced diameter portion 27 in order from the proximal end side to the distal end side. The expansion portion 25 is formed in the distal end portion of the small diameter portion 13. The expansion portion 25 has an enlarged diameter portion 25l, an intermediate large diameter portion 25m, and a reduced diameter portion 25n in order from the proximal end side to the distal end side. The enlarged diameter portion 25l is formed in the proximal end portion of the expansion portion 25. The enlarged diameter portion 25l is a portion having an outer diameter that gradually increases from the proximal end side to the distal end side. The intermediate large diameter portion 25m is formed between the enlarged diameter portion 25l and the reduced diameter portion 25n of the expansion portion 25. The intermediate large diameter portion 25m is a portion having an outer diameter that gradually increases toward the central position in the X-axis direction. The reduced diameter portion 25n is formed in the distal end portion of the expansion portion 25. The reduced diameter portion 25n is a portion having an outer diameter that gradually decreases from the proximal end side to the distal end side.

The distal end reduced diameter portion 27 is formed in the distal end portion of the expansion portion 25. In other words, the distal end reduced diameter portion 27 is formed in the distal end portion of the first magnetized region MR1. The distal end reduced diameter portion 27 is a portion having an outer diameter that gradually decreases from the proximal end side to the distal end side. Note that in the seventh embodiment, a dot pattern is formed by the hatching technique on the expansion portion 25 and the distal end reduced diameter portion 27, indicating the state of being magnetized. The distal end joint part 24f has a substantially sharp shape formed so as to cover the distal end reduced diameter portion 27, and joins the outer coil 30 and the distal end (the distal end of the distal end reduced diameter portion 27) of the core wire 10f.

Also with the guide wire 1F of the seventh embodiment as described above, it becomes possible for the distal end portion of the guide wire 1F to have a desired strength of magnetism, as in the case of the first embodiment. Further, the enlarged diameter portion 25l and the reduced diameter portion 25n make it difficult to create a rigidity gap, with which the bending rigidity is greatly varied in the distal end portion and the proximal end portion of the first magnetized region MR1, so as to be able to suppress the bending of the distal end portion and the proximal end portion of the first magnetized region MR1. Furthermore, the intermediate large diameter portion 25m can cause an increase in the volume of the first magnetized region MR1 per unit length. Moreover, in the seventh embodiment, the distal end portion of the first magnetized region MR1 may be formed with the distal end reduced diameter portion 27 having an outer diameter that gradually decreases from the proximal end side to the distal end side. Therefore, when there is a lesion or the like clogging a living body lumen into which the guide wire 1F is inserted, the seventh embodiment can facilitate the entry of the guide wire 1F into the lesion or the like. In the case of the seventh embodiment, the distal end joint part 24f, which is formed in a substantially sharp shape using the distal end reduced diameter portion 27 as a support, enters a lesion or the like, thereby facilitating the entry of the guide wire 1F into the lesion or the like.

### <Eighth to tenth embodiments>

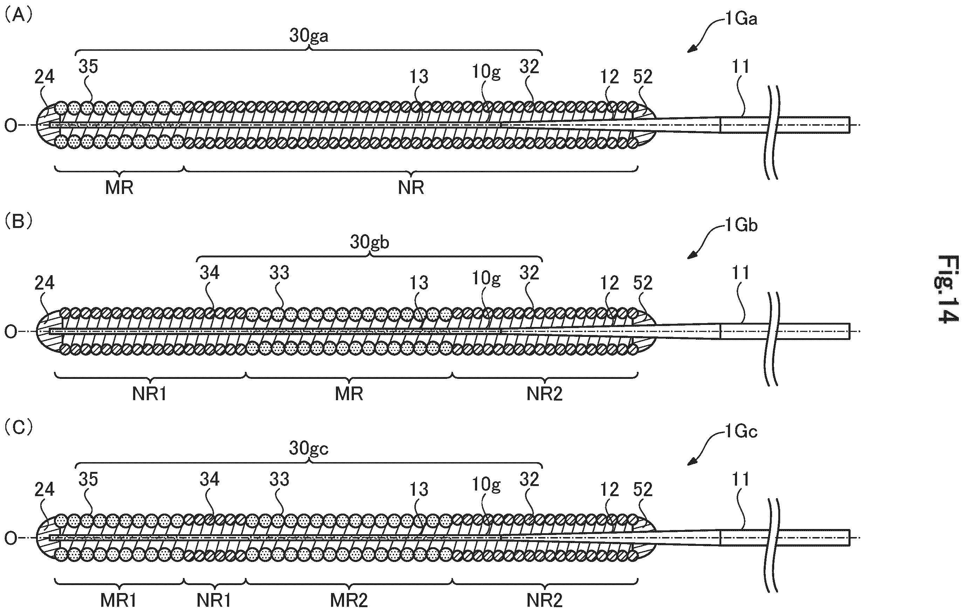

FIGS. 14A, 14B and 14C are explanatory views that schematically show the configurations of guide wires 1Ga, 1Gb and 1Gc of the eighth to tenth embodiments. The guide wires 1Ga, 1Gb and 1Gc of the eighth to tenth embodiments differ from the guide wire 1 (FIG. 1) of the first embodiment in that the guide wires 1Ga, 1Gb and 1Gc include a core wire 10g differing from the core wire 10 in the shape of the portion on the distal end side, and include outer coils 30ga, 30gb and 30gc, respectively, differing from the outer coil 30. The configurations of the guide wires 1Ga, 1Gb and 1Gc share a common feature of including the core wire 10g, but differ from each other in that the guide wires 1Ga, 1Gb and 1Gc include the outer coils 30ga, 30gb and 30gc, respectively.

First, the guide wire 1Ga of the eighth embodiment is described with reference to FIG. 14A. The core wire 10g has a large diameter portion 11, a reduced diameter portion 12, and a small diameter portion 13 in order from the proximal end side to the distal end side, but has no distal end large diameter portion 20 on the distal end side. The outer coil 30ga is a single-thread coil formed of a magnetic material that is magnetized by applying an external magnetic field, as in the case of the outer coil 30 of the first embodiment. Meanwhile, the outer coil 30ga is formed of wires 32 and 35 in order from the proximal end side to the distal end side, and the diameter of the wire 35 is larger than that of the wire 32. The outer coil 30ga is a coil formed by winding a single wire, which is formed of the wires 32 and 35 that are connected to each other to form a single thread. Note that, as shown in FIG. 14A, the outer coil 30ga has a substantially cylindrical shape (a shape having a substantially constant outer diameter of the transverse section).

The outer coil 30ga has a magnetized region MR magnetized on the distal end side. In the guide wire 1Ga, the portion of the core wire 10g arranged inside the magnetized region MR is also magnetized. Further, the outer coil 30ga has a non-magnetized region NR that is not magnetized and located to the proximal end side of the magnetized region MR. The magnetized region MR corresponds to a position where the wire 35 of the outer coil 30ga is wound. The non-magnetized region NR corresponds to a position where the wire 32 of the outer coil 30ga is wound. Of the outer coil 30ga, the wire 35 forming the magnetized region MR is thicker than the wire 32 forming the distal end portion in the non-magnetized region NR that is a region located to the proximal end side of the magnetized region MR. Also with the guide wire 1Ga of the eighth embodiment as described above, the volume of the magnetized region MR per unit length can be increased, so that the magnetized region MR can have strong magnetism. Moreover, the magnetized region MR corresponds to the distal end portion of the guide wire 1Ga, making it possible for the distal end portion of the guide wire 1Ga to have a desired strength of magnetism.

Next, the guide wire 1Gb of the ninth embodiment is described with reference to FIG. 14B. The outer coil 30gb is formed of wires 32, 33, and 34 in order from the proximal end side to the distal end side, and the diameter of the wire 33 is thicker than those of the wires 32 and 34. The outer coil 30gb is a coil formed by winding a single wire in a single thread, which is formed of the wires 32, 33 and 34 that are connected to each other. The outer coil 30gb has a magnetized region MR magnetized at the intermediate position. Also in the guide wire 1Gb, the portion of the core wire 10g arranged inside the magnetized region MR is also magnetized. Further, the outer coil 30gb has a first non-magnetized region NR1 that is not magnetized and located to the distal end side of the magnetized region MR, and has a second non-magnetized region NR2 that is not magnetized and located to the proximal end side of the magnetized region MR.

The magnetized region MR corresponds to the position where the wire 33 of the outer coil 30gb is wound. Moreover, the first non-magnetized region NR1 is located at a position where the wire 34 of the outer coil 30gb is wound, and the second non-magnetized region NR2 is located at a position where the wire 32 of the outer coil 30gb is wound. The wire 33 forming the magnetized region MR is thicker than the wire 32 forming the distal end portion in the second non-magnetized region NR2, and is thicker than the wire 34 forming the proximal end portion in the first non-magnetized region NR1. Also with the guide wire 1Gb of the ninth embodiment as described above, the volume of the magnetized region MR per unit length can be increased, so that the magnetized region MR can have strong magnetism.

Next, the guide wire 1Gc of the tenth embodiment is described with reference to FIG. 14C. The outer coil 30gc is formed of wires 32, 33, 34 and 35 in order from the proximal end side to the distal end side, and the diameters of the wires 33 and 35 are thicker than those of the wires 32 and 34. The outer coil 30gc is a coil formed by winding a single wire in a single thread, which is formed of the wires 32, 33 and 34 that are connected to each other. The outer coil 30gc has a first magnetized region MR1 magnetized on the distal end side. Further, the outer coil 30gc has the second magnetized region MR2 magnetized at a position that is located to the proximal end side of the first magnetized region MR1 and separated from the first magnetized region MR1. Also in the guide wire 1Gc, the portion of the core wire 10g arranged inside the first magnetized region MR1 and the second magnetized region MR2 is also magnetized. Moreover, in the outer coil 30gc, the first non-magnetized region NR1 is provided between the first magnetized region MR1 and the second magnetized region MR2, and a second non-magnetized region NR2 is provided to the proximal end side of the second magnetized region MR2.

The first magnetized region MR1 and the second magnetized region MR2 correspond to positions where the wires 35 and 33 of the outer coil 30gc are wound. On the other hand, the first non-magnetized region NR1 and the second non-magnetized region NR2 correspond to positions where the wires 34 and 32 of the outer coil 30gc are wound. The wire 35 forming the first magnetized region MR1 is thicker than the wire 34 forming the distal end portion in the first non-magnetized region NR1. The wire 33 forming the second magnetized region MR2 is thicker than the wire 34 forming the proximal end portion in the first non-magnetized region NR1, and is thicker than the wire 32 forming the distal end portion in the second non-magnetized region NR2. Also with the guide wire 1Gc of the tenth embodiment as described above, the volume of the first magnetized region MR1 and the second magnetized region MR2 per unit length can be increased, so that the first magnetized region MR1 and the second magnetized region MR2 can have strong magnetism.

In the eighth to tenth embodiments, the outer coils 30ga, 30gb, and 30gc are single-thread coils, but the outer coils 30ga, 30gb, and 30gc may be multi-thread coils, single-thread twisted wire coils, or multi-thread twisted wire coils, which are different from single-thread coils. In such a case, the diameter of the wire or twisted wire forming a portion corresponding to a position as the magnetized region MR or the position as the first (second) magnetized region MR1 (MR2) is formed to be thicker than the diameter of the wire or the twisted wire forming another portion(s), so that the same effects as those of the eighth to tenth embodiments can be exerted.

### <Modification examples of the embodiments>

The disclosed embodiments are not limited to those above, and can be implemented in various aspects without departing from the gist thereof. For example, the following modification examples are also possible.

### [Modification example 1]

In the first to tenth embodiments, the configurations of the guide wires 1, 1A to 1F, and 1Ga to Gc are illustrated. However, various modifications can be made to the configurations of the guide wires. For example, in the core wire included in the guide wire, the portion located to the proximal end side of the distal end large diameter portion may be a portion having a constant outer diameter, instead of a small diameter portion, a large diameter portion, and a reduced diameter portion. Further, the distal end large diameter portion as the first magnetized region may be formed on the distal end side excluding the distal end of the core wire 10. Also, the guide wire may have three or more magnetized regions. Further, the shape of the member forming the first magnetized region and the second magnetized region does not have to be substantially cylindrical, and may be a shape that spreads out so as to fill the inner space of the coil. Specifically, the shape of the same may be a shape having a projecting portion that fills a groove portion formed between wires wound as a coil. Moreover, the position of the outer coil and the same of the inner coil may be formed of a magnetic material at the positions where the external magnetic field is applied, and positions to which the external magnetic field is not applied may be formed of a non-magnetic material.

### [Modification example 2]

The configurations of the guide wires 1, 1A to 1F, and 1Ga to Gc of the first to tenth embodiments, and each configuration of the above modification example 1 may be combined appropriately. For example, in the guide wires 1 and 1A to 1C of the first to fourth embodiments, the portion where the outer diameter of the core wire changes (for example, the portion from the distal end of the small diameter portion 13 to the distal end large diameter portion 20) may be provided with a reduced diameter portion or an enlarged diameter portion. Further, the guide wires 1, 1C to 1F, and 1Ga to Gc of the first and fourth to tenth embodiments may have the curved portions described in the third embodiment. Further, in the guide wires 1C and 1D of the fourth and fifth embodiments, the inner coil described in the second embodiment and the like may cover the outer peripheral surfaces of the intermediate large diameter portions 14 and 14d. Further, in the guide wires 1E and 1F of the sixth and seventh embodiments, at least one of the inner coil described in the second embodiment and the like and the intermediate large diameter portion described in the fourth embodiment and the like may be provided. Further, the guide wires 1Ga to Gc of the eighth to tenth embodiments may include the core wires 10 and 10c to 10f of the first and fourth to seventh embodiments instead of the core wire 10g, or may include the core wire 10 having an outer peripheral surface that is covered with the inner coil 40 of the second embodiment.

The present aspects are described above on the basis of the embodiments and the modification examples. However, the embodiments of the aforementioned aspects are provided to facilitate the understanding of the present aspects, and do not limit the present aspects. The present aspects may be altered or improved without departing from the spirit thereof and claims, and the present aspects include their equivalents. Further, the technical features thereof, if not indicated as essential in the present specification, may be appropriately deleted.

### [Reference Signs List]

1, 1Ato F and 1Ga to Gc: guide wire
10 and 10b to g: core wire
10p1 to 4: rod-shaped member
11: large diameter portion
12: reduced diameter portion
13: small diameter portion
14 and 14d: intermediate large diameter portion
15: distal end side small diameter portion
20, 20d and 20e: distal end large diameter portion
20p: cylindrical portion
21: constricted portion
21l: reduced diameter portion
21m: intermediate small diameter portion
21n: enlarged diameter portion
23: enlarged diameter portion
24 and 24f: distal end joint part
25: expansion portion
25l: enlarged diameter portion
25m: intermediate large diameter portion
25n: reduced diameter portion
27: distal end reduced diameter portion
30 and 30ga to gc: outer coil
31, 32, 33, 34 and 35: wire
40: inner coil
41: wire
42: proximal joint part
44: distal joint part
42p: joint part
44p: joint part
52: proximal end joint part
CV: curved portion
MR1: first magnetized region
MR2: second magnetized region
NR1: first non-magnetized region
NR2: second non-magnetized region
O: axis
r1 to 8: outer diameter

## Claims

1. A guide wire, comprising a core wire, wherein the core wire has a first magnetized region magnetized on the distal end side of the core wire, and the maximum value of the outer diameter of the first magnetized region is larger than the outer diameter of the distal end portion in an area located to the proximal end side of the first magnetized region.

2. The guide wire according to claim 1, wherein the core wire further has a second magnetized region magnetized at a position that is located to the proximal end side of the first magnetized region and separated from the first magnetized region, and the maximum value of the outer diameter of the first magnetized region is larger than the outer diameter of the distal end portion in an area between the first magnetized region and the second magnetized region.

3. The guide wire according to claim 2, wherein the second magnetized region comprises a magnetized coil that covers the outer peripheral surface of the core wire, and the maximum value of the outer diameter of the coil as the second magnetized region is larger than the outer diameter of the proximal end portion in the area between the first magnetized region and the second magnetized region, and larger than the outer diameter of the distal end portion in an area located to the proximal end side of the second magnetized region.

4. The guide wire according to claim 2, wherein the core wire is formed so that the maximum value of the outer diameter of the core wire in the second magnetized region is larger than the outer diameter of the proximal end portion in the area between the first magnetized region and the second magnetized region, and larger than the outer diameter of the distal end portion in the area located to the proximal end side of the second magnetized region.

5. The guide wire according to any one of claims 2 to 4, wherein the core wire has a curved portion in which the core wire is curved in the area between the first magnetized region and the second magnetized region.

6. The guide wire according to any one of claims 1 to 5, wherein the proximal end portion of the first magnetized region is formed with an enlarged diameter portion having an outer diameter that gradually increases from the proximal end side to the distal end side.

7. The guide wire according to any one of claims 1 to 6, wherein the proximal end portion of the first magnetized region is formed with a distal end reduced diameter portion having an outer diameter that gradually decreases from the proximal end side to the distal end side.

8. A guide wire, comprising a core wire and a coil covering the core wire, wherein the coil has a magnetized region that is magnetized, and a wire forming the magnetized region of the coil is thicker than a wire forming the distal end portion in an area located to the proximal end side of the magnetized region.
